# EUROPEAN PATENT SPECIFICATION

(11) **EP 4 279 479 B1**
(45) Date of publication and mention of the grant of the patent: **12.08.2026**
(21) Application number: 22898983.6
(22) Date of filing: 22.11.2022
(51) Int. Cl.: C07C 68/06, C07C 69/96

(54) **ASYMMETRIC LINEAR CARBONATE AND METHOD FOR PREPARING ASYMMETRIC LINEAR CARBONATE**
ASYMMETRISCHES LINEARES CARBONAT UND VERFAHREN ZUR HERSTELLUNG VON ASYMMETRISCHEM LINEAREM CARBONAT
CARBONATE LINÉAIRE ASYMÉTRIQUE ET PROCÉDÉ DE PRÉPARATION DE CARBONATE LINÉAIRE ASYMÉTRIQUE

(30) Priority: 23.11.2021 KR 20210162578; 17.11.2022 KR 20220154684
(43) Date of publication of application: 22.11.2023
(73) Proprietor: LG Chem, Ltd., Seoul 07336 (KR)
(72) Inventor: LEE, Hyunyoung, Daejeon 34122 (KR); PARK, Jun Beom, Daejeon 34122 (KR); KIM, Hyun Cheol, Daejeon 34122 (KR)
(74) Representative: Goddar, Heinz J.
(86) International application number: PCT/KR2022/018473
(87) International publication number: WO 2023/096301

(56) References cited:
- CN-A- 107 513 017
- JP-A- H0 959 224
- JP-A- H0 959 224
- JP-A- H10 338 663
- KR-A- 20000 051 813
- KR-A- 20000 051 813
- KR-A- 20060 054 533
- KR-A- 980 002 000

## Description

### FIELD OF THE INVENTION

The present invention relates to an asymmetric linear carbonate and a method for preparing the asymmetric linear carbonate.

### BACKGROUND

Since electrolyte solvents for lithium ion batteries must be dissolved well in lithium ions and move smoothly, it is required to have high salt solubility and low viscosity. Thus, cyclic carbonates (ethylene carbonate, propylene carbonate, etc.) dissolving well in salts, and linear carbonates (ethylmethyl carbonate, dimethyl carbonate, diethyl carbonate, etc.) having low viscosity are mixed and used.

Particularly, ethyl methyl carbonate (EMC), which is an asymmetric linear carbonate, is superior to other solvents in terms of energy storage density, charge capacity, charge/discharge recovery, stability and the like and thus, is the most preferred solvent.

As a method for preparing such ethyl methyl carbonate, there is known a method that utilizes an ester reaction between alkyl chloroformate and alcohol in the presence of a basic catalyst, but this method has a problem that the esterification reaction is very vigorous and that toxic compounds such as phosgene and bisphenol-A must be used as starting materials.

In order to compensate for these problems, a method that utilizes a transesterification reaction of a symmetrical linear carbonate and an alcohol are known, but this has a problem that an asymmetric linear carbonate such as ethyl methyl carbonate must be separated and purified from a reaction product containing three kinds of linear carbonates and two kinds of alcohols through a complicated process.

Therefore, there is a need to develop a method for preparing ethyl methyl carbonate having highly added value, so that the ethyl methyl carbonate can be easily separated and purified.

KR 2000 0051813 A discloses the transesterification of two different types of symmetrical linear carbonic acid esters utilizing a group IA element in an alkoxide salt catalyst.

JP H 0959224 A discloses transition metal alkoxides or halides such as iron, tin, cobalt or vanadium as transesterification catalysts.

CN 107513017 A discloses the use of calcium methoxide as transesterification catalyst.

### SUMMARY OF THE INVENTION

### TECHNICAL PROBLEM

It is an object of the present invention to provide a method for preparing an asymmetric linear carbonate in which a transesterification process can proceed by a solvent-free process, thereby permitting each separation and purification of an asymmetric linear carbonate having a highly added value from the final reactant, and a high-purity asymmetric linear carbonate prepared using such a preparation method.

### TECHNICAL SOLUTION

Provided herein is a method for preparing an asymmetric linear carbonate, the method comprising: subjecting two kinds of different symmetric linear carbonates to a transesterification reaction in the presence of a metal alkoxide catalyst to prepare an asymmetric linear carbonate, wherein the metal of the metal alkoxide is at least one selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V).

Also provided herein is an asymmetric linear carbonate comprising a metal alkoxide catalyst which includes at least one metal selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V).

Further embodiments are disclosed in the dependent claims.

### DETAILED DESCRIPTION

Hereinafter, an asymmetric linear carbonate and a method for preparing an asymmetric linear carbonate according to specific embodiment of the present invention is described in detail.

Unless explicitly stated herein, the technical terms is for the purpose of describing specific embodiments only and is not intended to limit the scope of the invention.

The singular forms "a," "an" and "the" are intended to include plural forms, unless the context clearly indicates otherwise.

It should be understood that the terms "comprise," "include", "have", etc. are used herein to specify the presence of stated features, regions, integers, steps, actions, elements and/or components, but do not preclude the presence or addition of one or more other features, regions, integers, steps, actions, elements, components and/or groups.

Further, the steps constituting the preparation method described herein are not construed as being limited to the order in which one step and the other steps constituting one preparation method are described herein, unless explicitly stated as being sequential or continuous order or otherwise specified. Therefore, the order of the constituent steps of the preparation method can be changed within a range that can be easily understood by those skilled in the art, and in this case, changes apparent to those skilled in the art accompanying therewith are included in the scope of the invention.

According to specific embodiment of the present invention, provided is a method for preparing an asymmetric linear carbonate, the method comprising: subjecting two kinds of different symmetric linear carbonates to a transesterification reaction in the presence of a metal alkoxide catalyst to prepare an asymmetric linear carbonate, wherein the metal of the metal alkoxide is at least one selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V).

The present inventors conducted intensive research on a method for preparing symmetric linear carbonates such as ethyl methyl carbonate, and found through experiments that when an asymmetric linear carbonate is prepared by subjecting two kinds of different symmetric linear carbonates to a transesterification reaction in the presence of a metal alkoxide catalyst which includes at least one metal selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V), the transesterification reaction is performed by a solvent-free process to prepare a large amount of asymmetric linear carbonate, and most of the three types of linear carbonates including two different symmetric linear carbonates and asymmetric linear carbonates are included in the final reactant, which permits easy separation and purification of the asymmetric linear carbonate, and completed the present invention.

When the transesterification reaction is performed under a metal alkoxide catalyst containing alkali metals such as lithium and sodium, solvents such as methanol and ethanol are further formed in addition to the three linear carbonates in the final product, which causes a problem that the asymmetric linear carbonate, which is the final target compound, must be separated and purified by a more complicated process.

However, the metal alkoxide catalyst used in the preparation method of asymmetric linear carbonate according to the one embodiment includes at least one metal selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V), and a transesterification reaction can be performed under such a metal alkoxide catalyst.

In the case of a conventional metal alkoxide catalyst containing alkali metals such as lithium and sodium, for example, in the case of sodium ethoxide, it is not dissolved in a non-polar solvent without a polar solvent such as ethanol, and thus essentially requires a polar solvent such as ethanol during the progress of the transesterification reaction.

Meanwhile, in the case of the metal alkoxide catalyst, for example, aluminum ethoxide or magnesium ethoxide, used in the preparation method of an asymmetric linear carbonate according to one embodiment, it is easily dissolved in linear carbonates such as dimethyl carbonate (DMC) and diethyl carbonate (DEC) and thus does not require polar solvents such as ethanol, so that it contains significantly less alcoholic solvents such as methanol and ethanol as compared to the case of using sodium ethoxide and the like in the final reaction product, thereby easily separating and purifying the asymmetric linear carbonate from the final reaction product.

In particular, the method for preparing an asymmetric linear carbonate has a low ratio of highly azeotropic alcohol in the final reactant, thereby permitting easy recovery of the asymmetric linear carbonate through distillation in a later process.

The two kinds of different symmetrical linear carbonates can be two selected from the group consisting of dimethyl carbonate, diethyl carbonate, dipropyl carbonate and dibutyl carbonate, and can be, for example, dimethyl carbonate and diethyl carbonate.

In addition, the asymmetric linear carbonate can be ethyl methyl carbonate, methyl propyl carbonate, ethyl propyl carbonate, butyl methyl carbonate, butyl ethyl carbonate or butyl propyl carbonate.

Moreover, as the transesterification reaction is performed under the metal alkoxide catalyst, it can proceed by a solvent-free process. Accordingly, the final reactant hardly contains other solvents, and most thereof contain two kinds of different symmetric linear carbonates and a reactant asymmetric linear carbonate, thereby easily performing separation and purification of asymmetric linear carbonates from these final reactants.

In particular, the method for preparing an asymmetric linear carbonate has a low ratio of highly azeotropic alcohol in the final reactant, thereby performing easy recovery of the asymmetric linear carbonate through distillation in a later process.

The metal alkoxide catalyst can be represented by the following Chemical Formula 1:

[Chemical Formula 1] X(O-R)a

wherein, in Chemical Formula 1:
X is at least one selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V);
R is an alkyl group having 1 to 10 carbon atoms; and
a is an integer of 2 or more.

Further, R can be ethyl, methyl, n-propyl, isopropyl, n-butyl, isobutyl, tert-butyl, pentyl, tert-pentyl, neopentyl, isopentyl or sec-pentyl.

Alternatively, a can be an integer of 2 or more and 10 or less.

An example of the metal alkoxide catalyst can be at least one selected from the group consisting of aluminum trimethoxide, aluminum triethoxide, aluminum isopropoxide, aluminum tert-butoxide, magnesium dimethoxide, magnesium diethoxide, magnesium isopropoxide, magnesium tert-butoxide and calcium methoxide.

The metal alkoxide catalyst can be included in an amount of 0.1 parts by weight or more, 0.2 parts by weight or more, 0.3 parts by weight or more, 0.5 parts by weight or more, and 10.0 parts by weight or less, 8.0 parts by weight or less, 6.0 parts by weight or less, or 5.0 parts by weight or less, based on 100 parts by weight of the two kinds of different symmetrical linear carbonates.

Even if the content of the metal alkoxide catalyst exceeds 10.0 parts by weight, the reaction is not additionally activated, which is uneconomical and inefficient. If the content of the base catalyst of the heterocyclic structure is too small, the transesterification reaction rate can decrease.

Meanwhile, in the method for preparing an asymmetric linear carbonate, the two kinds of different symmetrical linear carbonates can be dimethyl carbonate and diethyl carbonate, and the molar ratio of dimethyl carbonate and diethyl carbonate can be 1:0.5 to 1:1.5, 1:0.7 to 1:1.3, and 1:0.9 to 1:1.1. If the content of diethyl carbonate relative to dimethyl carbonate is too low or too high, the conversion rate to the finally produced ethyl methyl carbonate can be remarkably low.

In the method for preparing an asymmetric linear carbonate of one embodiment, the transesterification reaction can be performed at a temperature of 70°C or more, 80°C or more, 90°C or more, 100°C or more, 110°C or more, or can be performed at a temperature of 150°C or less, 140°C or less, 130°C or less.

Further, the transesterification reaction can be performed for 1 hour or more, 2 hours or more, 3 hours or more, or can be performed for 12 hours or less, 11 hours or less, 10 hours or less, or 8 hours or less.

Further, the transesterification reaction can be performed under atmospheric pressure conditions of 1 atm or more, 2 atm or more, or 3 atm or more, or can be performed under atmospheric pressure conditions of 10 atm or less, 9 atm or less, 8 atm or less, or 7 atm or less (1 atm = 101325 Pa).

Further, the method for preparing an asymmetric linear ester according to the one embodiment can further include recovering the asymmetric linear carbonate.

After the transesterification reaction is completed, preferably three kinds of linear carbonates can be present in the reaction product. For example, two different symmetric linear carbonates and asymmetric linear carbonates can be included. In addition, the reaction product contains a significantly small amount of alcohol solvent, so that the asymmetric linear carbonate can be easily separated and purified from the final reaction product.

For example, the asymmetric linear carbonate can be separated from the reaction product by atmospheric or under reduced pressure distillation. When the reaction product is distilled under atmospheric pressure or reduced pressure, distillation starts from the compound with the lowest boiling point to the compound with the highest boiling point, and finally, asymmetric linear carbonates having a high purity can be recovered.

For example, when dimethyl carbonate, diethyl carbonate, ethyl methyl carbonate are contained in the reaction product, the reaction products can be separated in order of dimethyl carbonate (boiling point: 90°C), ethylmethyl carbonate, and diethyl carbonate (boiling point: 127°C) during purification thereof to recover ethyl methyl carbonates having a high purity of 80% or more, 85% or more, 90% or more or 99.9% or more. At this time, the separated dimethyl carbonate and diethyl carbonate can be recovered and reused.

According to another embodiment of the present invention, provided is an asymmetric linear carbonate comprising a metal alkoxide catalyst which includes at least one metal selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V).

The metal alkoxide catalyst can be represented by the following Chemical Formula 1:

[Chemical Formula 1] X(O-R)a

wherein, in Chemical Formula 1:
X is at least one selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V);
R is an alkyl group having 1 to 10 carbon atoms; and
a is an integer of 2 or more.

Further, R can also be ethyl, methyl, n-propyl, isopropyl, n-butyl, isobutyl, tert-butyl, pentyl, tert-pentyl, neopentyl, isopentyl or sec-pentyl.

Alternatively, a can be an integer of 2 or more and 10 or less.

One example of the metal alkoxide catalyst can be at least one selected from the group consisting of aluminum trimethoxide, aluminum triethoxide, aluminum isopropoxide, aluminum tert-butoxide, magnesium dimethoxide, magnesium diethoxide, magnesium isopropoxide, magnesium tert-butoxide and calcium methoxide.

In addition, the asymmetric linear carbonate can be ethyl methyl carbonate, methyl propyl carbonate, ethyl propyl carbonate, butyl methyl carbonate, butyl ethyl carbonate or butyl propyl carbonate.

The metal alkoxide catalyst can be included in an amount of 0.01 parts by weight or more, 0.05 parts by weight or more, 0.10 parts by weight or more, or 0.50 parts by weight or more, and 5.00 parts by weight or less, 3.00 parts by weight or less, or 1.00 parts by weight or less, based on 100 parts by weight of the asymmetric linear carbonate.

### ADVANTAGEOUS EFFECTS

The present invention can provide an asymmetric linear carbonate having a high purity and highly added value at a high conversion rate, and a method for preparing an asymmetric linear carbonate that permits each separation and purification of an asymmetric linear carbonate having a highly added value from a final reactant by proceeding a transesterification reaction by a solvent-free process, and allows easy process control and mass production.

### EXAMPLES

Hereinafter, the present invention will be described in more detail by way of examples. However, these examples are merely presented for illustrative purposes only, and the scope of the invention is not determined thereby.

### Example 1

A 100 mL flask was charged with 1.0 mol of dimethyl carbonate (DMC), 1.0 mol of diethyl carbonate (DEC), and 0.3 wt% of an aluminum triethoxide (Al(OEt)₃) catalyst in powder form. Then, the temperature inside the flask was raised to 120°C and the mixture was reacted for 3 hours.

### Example 2

Ethyl methyl carbonate was prepared in the same manner as in Example 1, except that 0.3 wt.% of magnesium diethoxide (Mg(OEt)₂) catalyst in powder form was used instead of 0.3 wt.% of aluminum triethoxide (Al(OEt)₃) catalyst.

### Comparative Example 1

Ethyl methyl carbonate was prepared in the same manner as in Example 1, except that 0.3 wt.% of sodium ethoxide (NaOEt) catalyst in powder form was used instead of 0.3 wt.% of aluminum triethoxide (Al(OEt)₃) catalyst.

### Comparative Example 2

Ethyl methyl carbonate was prepared in the same manner as in Example 1, except that 1.5 wt.% of a solution in which sodium ethoxide (NaOEt) catalyst was dissolved at a concentration of 20% (NaOEt 20% in EtOH) was used instead of 0.3 wt.% of aluminum triethoxide (Al(OEt)₃) catalyst.

### Comparative Example 3

Ethyl methyl carbonate was prepared in the same manner as in Example 1, except that 1.5 wt.% of a solution (NaOEt 20% in EtOH) in which liquid titanium tetraethoxide (Ti(OEt)₄) was dissolved at a concentration of 20% was used instead of 0.3 wt.% of aluminum triethoxide (Al(OEt)₃) catalyst.

### <Experimental Example>

### 1. Gas chromatograph (GC) analysis results

After transesterification reaction of Examples and Comparative Examples, gas chromatograph (GC) analysis was performed to confirm the products and residues. The results are shown in Table 1 below.

**[Table 1]**

| | MeOH (mol%) | EtOH (mol%) | DMC (mol%) | EMC (mol%) | DEC (mol%) |
|---|---|---|---|---|---|
| Example 1 | 3.0 | 0.0 | 27.3 | 38.1 | 31.6 |
| Example 2 | 3.1 | 0.0 | 22.9 | 48.7 | 25.2 |
| Comparative Example 1 | 4.2 | 0.7 | 45.2 | 0.0 | 50.0 |
| Comparative Example 2 | 4.2 | 0.7 | 20.6 | 47.8 | 26.8 |
| Comparative Example 3 | 5.3 | 0.9 | 35.0 | 12.6 | 46.2 |

Referring to Table 1, it can be seen that Examples 1 and 2 using aluminum triethoxide and magnesium diethoxide as catalysts, respectively, hardly formed methanol (MeOH) and ethanol (EtOH), which are other solvents, in addition to DMC, EMC and DEC, which permitted easy recovery of EMC therefrom.

On the other hand, it was confirmed that in Comparative Example 1 using the sodium ethoxide catalyst in powder form, both methanol (MeOH) and ethanol (EtOH) were produced, but EMC was not produced at all. It can be expected that in Comparative Example 2 using a solution in which sodium ethoxide catalyst was dissolved, EMC was produced, but more methanol (MeOH) and ethanol (EtOH) were formed than Examples 1 and 2, which made it difficult to recover EMC therefrom. In addition, it was confirmed that in Comparative Example 3 using the titanium tetraethoxide catalyst, not only methanol (MeOH) and ethanol (EtOH) were formed, but also EMC was prepared as low as 12.6 mol%.

### 2. Ion analysis results

After the transesterification reaction of Examples and Comparative Examples, analysis was performed by inductively coupled plasma mass spectrometry (ICP-MS) and combustion ion chromatography (C-IC) to confirm products and residues, and the results are shown in Tables 2 and 3 below.

**[Table 2]**

| (unit: ppb) | Example 1 | Example 2 | Comparative Example 1 | Comparative Example 2 | Comparative Example 3 |
|---|---|---|---|---|---|
| Na | 4 | 3 | 32414 | 67661 | 4 |
| Mg | 4 | 452 | <1 | 4 | 3 |
| Al | 902 | 12 | 1 | 12 | 10 |
| K | 15 | 4 | 40 | 56 | 4 |
| Ca | 26 | 4 | 12 | 17 | 5 |
| Ti | <1 | <1 | <1 | <1 | 9753 |
| Cr | 5 | 4 | 110 | 57 | 4 |
| Mn | <1 | <1 | <1 | <1 | <1 |
| Fe | 3 | <1 | <1 | <1 | 4 |
| Co | <1 | <1 | <1 | <1 | <1 |
| Zn | 2 | <1 | <1 | <1 | 1 |
| Zr | <1 | <1 | <1 | <1 | <1 |
| Mo | 1 | <1 | 12 | 5 | <1 |
| Ba | <1 | <1 | <1 | <1 | <1 |
| Pb | <1 | <1 | <1 | <1 | <1 |

**[Table 3]**

| | Example 1 | Example 2 | Comparative Example 1 | Comparative Example 2 | Comparative Example 3 |
|---|---|---|---|---|---|
| Cl | <2 | <2 | 6 | 5 | <2 |
| Br | <2 | <2 | <2 | <2 | <2 |
| SO₄ | 4 | 5 | 4 | 4 | 6 |

Referring to Tables 2 and 3, it was confirmed that in Examples 1 and 2, the most included metal ion impurities were aluminum and magnesium, respectively, and their contents were 902 ppb or less, whereas in Comparative Examples 1 to 3, the contents of sodium and titanium, which are metal ion impurities, were 32,414 ppb, 67,661 ppb, and 9,753 ppb, respectively, so that that Comparative Examples 1 to 3 contained significantly more metal ion impurities than Examples 1 and 2. In addition, it can be predicted that in Comparative Examples 1 to 3 having a large content of such metal ion impurities, the purification of ethyl methyl carbonate was difficult.

## Claims

1. A method for preparing an asymmetric linear carbonate, the method comprising:
subjecting two kinds of different symmetric linear carbonates to a transesterification reaction in the presence of a metal alkoxide catalyst to prepare an asymmetric linear carbonate,
wherein the metal of the metal alkoxide is at least one selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V).

2. The method for preparing an asymmetric linear carbonate according to claim 1, wherein:
the two kinds of different symmetrical linear carbonates are dimethyl carbonate and diethyl carbonate, and
the asymmetric linear carbonate is ethyl methyl carbonate.

3. The method for preparing an asymmetric linear carbonate according to claim 1, wherein:
the transesterification reaction is performed by a solvent-free process.

4. The method for preparing an asymmetric linear carbonate according to claim 1, wherein:
the metal alkoxide catalyst is represented by the following Chemical Formula 1:
[Chemical Formula 1] X(O-R)a
wherein, in Chemical Formula 1,
X is at least one selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V).
R is an alkyl group having 1 to 10 carbon atoms, and
a is an integer of 2 or more.

5. The method for preparing an asymmetric linear carbonate according to claim 1, wherein:
the metal alkoxide catalyst is at least one selected from the group consisting of aluminum trimethoxide, aluminum triethoxide, aluminum isopropoxide, aluminum tert-butoxide, magnesium dimethoxide, magnesium diethoxide, magnesium isopropoxide, magnesium tert-butoxide and calcium methoxide.

6. The method for preparing an asymmetric linear carbonate according to claim 1, wherein:
a content of the metal alkoxide catalyst is 0.1 parts by weight or more and 10 parts by weight or less based on 100 parts by weight of the two kinds of different symmetrical linear carbonates.

7. The method for preparing an asymmetric linear carbonate according to claim 1, wherein:
the transesterification reaction is performed at a temperature of 70°C or more and 150°C or less.

8. The method for preparing an asymmetric linear carbonate according to claim 2, wherein:
a molar ratio of the dimethyl carbonate and diethyl carbonate is 1:0.5 to 1:1.5.

9. The method for preparing an asymmetric linear carbonate according to claim 1, further comprising recovering the asymmetric linear carbonate.

10. An asymmetric linear carbonate, comprising a metal alkoxide catalyst which includes at least one metal selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V).

11. The asymmetric linear carbonate according to claim 10, wherein:
the metal alkoxide catalyst is represented by the following Chemical Formula 1:
[Chemical Formula 1] X(O-R)a
wherein, in Chemical Formula 1:
X is at least one selected from the group consisting of aluminum (Al), magnesium (Mg), germanium (Ge), gallium (Ga), cobalt (Co), calcium (Ca), hafnium (Hf), iron (Fe), nickel (Ni), niobium (Nb), molybdenum (Mo), lanthanum (La), rhenium (Re), scandium (Sc), silicon (Si), tantalum (Ta), tungsten (W), yttrium (Y), zirconium (Zr) and vanadium (V);
R is an alkyl group having 1 to 10 carbon atoms; and
a is an integer of 2 or more.

12. The asymmetric linear carbonate according to claim 10, wherein:
the metal alkoxide catalyst is at least one selected from the group consisting of aluminum trimethoxide, aluminum triethoxide, aluminum isopropoxide, aluminum tert-butoxide, magnesium dimethoxide, magnesium diethoxide, magnesium isopropoxide, magnesium tert-butoxide and calcium methoxide.

13. The asymmetric linear carbonate according to claim 10, wherein:
a content of the metal alkoxide catalyst is 0.01 parts by weight or more and 5 parts by weight or less based on 100 parts by weight of the two kinds of different symmetrical linear carbonates.

14. The asymmetric linear carbonate according to claim 10, wherein:
the asymmetric linear carbonate is ethyl methyl carbonate.

## Patentansprüche

1. Verfahren zur Herstellung eines asymmetrischen linearen Carbonats, wobei das Verfahren umfasst:
Unterziehen von zwei Arten von verschiedenen symmetrischen linearen Carbonaten einer Umesterungsreaktion in Gegenwart eines Metallalkoxidkatalysators, um ein asymmetrisches lineares Carbonat herzustellen,
wobei das Metall des Metallalkoxids mindestens eines ist, ausgewählt aus der Gruppe bestehend aus Aluminium (AI), Magnesium (Mg), Germanium (Ge), Gallium (Ga), Kobalt (Co), Kalzium (Ca), Hafnium (Hf), Eisen (Fe), Nickel (Ni), Niob (Nb), Molybdän (Mo), Lanthan (La), Rhenium (Re), Scandium (Sc), Silizium (Si), Tantal (Ta), Wolfram (W), Yttrium (Y), Zirkonium (Zr) und Vanadium (V).

2. Verfahren zur Herstellung eines asymmetrischen linearen Carbonats nach Anspruch 1, wobei:
die zwei Arten von verschiedenen symmetrischen linearen Carbonaten Dimethylcarbonat und Diethylcarbonat sind, und
das asymmetrische lineare Carbonat Ethylmethylcarbonat ist.

3. Verfahren zur Herstellung eines asymmetrischen linearen Carbonats nach Anspruch 1, wobei:
die Umesterungsreaktion durch ein lösungsmittelfreies Verfahren durchgeführt wird.

4. Verfahren zur Herstellung eines asymmetrischen linearen Carbonats nach Anspruch 1, wobei:
der Metallalkoxidkatalysator durch die folgende chemische Formel 1 dargestellt wird:
[Chemische Formel 1] X(O-R)a
wobei in der chemischen Formel 1
X mindestens eines ist, ausgewählt aus der Gruppe bestehend aus Aluminium (AI), Magnesium (Mg), Germanium (Ge), Gallium (Ga), Kobalt (Co), Kalzium (Ca), Hafnium (Hf), Eisen (Fe), Nickel (Ni), Niob (Nb), Molybdän (Mo), Lanthan (La), Rhenium (Re), Scandium (Sc), Silizium (Si), Tantal (Ta), Wolfram (W), Yttrium (Y), Zirkonium (Zr) und Vanadium (V),
R eine Alkylgruppe mit 1 bis 10 Kohlenstoffatomen ist, und
a eine ganze Zahl von 2 oder mehr ist.

5. Verfahren zur Herstellung eines asymmetrischen linearen Carbonats nach Anspruch 1, wobei:
der Metallalkoxidkatalysator mindestens einer ist, ausgewählt aus der Gruppe bestehend aus Aluminiumtrimethoxid, Aluminiumtriethoxid, Aluminiumisopropoxid, Aluminium-tert-butoxid, Magnesiumdimethoxid, Magnesiumdiethoxid, Magnesiumisopropoxid, Magnesium-tert-butoxid und Kalziummethoxid.

6. Verfahren zur Herstellung eines asymmetrischen linearen Carbonats nach Anspruch 1, wobei:
ein Gehalt des Metallalkoxidkatalysators 0,1 Gewichtsteile oder mehr und 10 Gewichtsteile oder weniger beträgt, bezogen auf 100 Gewichtsteile der zwei Arten von verschiedenen symmetrischen linearen Carbonaten.

7. Verfahren zur Herstellung eines asymmetrischen linearen Carbonats nach Anspruch 1, wobei:
die Umesterungsreaktion bei einer Temperatur von 70 °C oder mehr und 150 °C oder weniger durchgeführt wird.

8. Verfahren zur Herstellung eines asymmetrischen linearen Carbonats nach Anspruch 2, wobei:
ein Molverhältnis des Dimethylcarbonats und Diethylcarbonats 1:0,5 bis 1:1,5 beträgt.

9. Verfahren zur Herstellung eines asymmetrischen linearen Carbonats nach Anspruch 1, ferner umfassend das Gewinnen des asymmetrischen linearen Carbonats.

10. Asymmetrisches lineares Carbonat, umfassend einen Metallalkoxidkatalysator, der mindestens ein Metall enthält, ausgewählt aus der Gruppe bestehend aus Aluminium (AI), Magnesium (Mg), Germanium (Ge), Gallium (Ga), Kobalt (Co), Kalzium (Ca), Hafnium (Hf), Eisen (Fe), Nickel (Ni), Niob (Nb), Molybdän (Mo), Lanthan (La), Rhenium (Re), Scandium (Sc), Silizium (Si), Tantal (Ta), Wolfram (W), Yttrium (Y), Zirkonium (Zr) und Vanadium (V).

11. Asymmetrisches lineares Carbonat nach Anspruch 10, wobei:
der Metallalkoxidkatalysator durch die folgende chemische Formel 1 dargestellt wird:
[Chemische Formel 1] X(O-R)a
wobei in der chemischen Formel 1:
X mindestens eines ist, ausgewählt aus der Gruppe bestehend aus Aluminium (AI), Magnesium (Mg), Germanium (Ge), Gallium (Ga), Kobalt (Co), Kalzium (Ca), Hafnium (Hf), Eisen (Fe), Nickel (Ni), Niob (Nb), Molybdän (Mo), Lanthan (La), Rhenium (Re), Scandium (Sc), Silizium (Si), Tantal (Ta), Wolfram (W), Yttrium (Y), Zirkonium (Zr) und Vanadium (V);
R eine Alkylgruppe mit 1 bis 10 Kohlenstoffatomen ist; und
a eine ganze Zahl von 2 oder mehr ist.

12. Asymmetrisches lineares Carbonat nach Anspruch 10, wobei:
der Metallalkoxidkatalysator mindestens einer ist, ausgewählt aus der Gruppe bestehend aus Aluminiumtrimethoxid, Aluminiumtriethoxid, Aluminiumisopropoxid, Aluminium-tert-butoxid, Magnesiumdimethoxid, Magnesiumdiethoxid, Magnesiumisopropoxid, Magnesium-tert-butoxid und Kalziummethoxid.

13. Asymmetrisches lineares Carbonat nach Anspruch 10, wobei:
ein Gehalt des Metallalkoxidkatalysators 0,01 Gewichtsteile oder mehr und 5 Gewichtsteile oder weniger beträgt, bezogen auf 100 Gewichtsteile der zwei Arten von verschiedenen symmetrischen linearen Carbonaten.

14. Asymmetrisches lineares Carbonat nach Anspruch 10, wobei:
das asymmetrische lineare Carbonat Ethylmethylcarbonat ist.

## Revendications

1. Procédé de préparation d'un carbonate linéaire asymétrique, le procédé consistant à :
soumettre deux sortes de carbonates linéaires symétriques différents à une réaction de transestérification en présence d'un catalyseur d'alcoxyde métallique pour préparer un carbonate linéaire asymétrique,
dans lequel le métal de l'alcoxyde métallique est un ou plusieurs éléments sélectionnés dans le groupe constitué d'aluminium (AI), magnésium (Mg), germanium (Ge), gallium (Ga), cobalt (Co), calcium (Ca), hafnium (Hf), fer (Fe), nickel (Ni), niobium (Nb), molybdène (Mo), lanthane (La), rhénium (Re), scandium (Sc), silicium (Si), tantale (Ta), tungstène (W), yttrium (Y), zirconium (Zr) et vanadium (V).

2. Procédé de préparation d'un carbonate linéaire asymétrique selon la revendication 1, dans lequel :
les deux sortes de carbonates linéaires symétriques différents sont le carbonate de diméthyle et le carbonate de diéthyle, et
le carbonate linéaire asymétrique est le carbonate d'éthylméthyle.

3. Procédé de préparation d'un carbonate linéaire asymétrique selon la revendication 1, dans lequel :
la réaction de transestérification est effectuée par un processus exempt de solvant.

4. Procédé de préparation d'un carbonate linéaire asymétrique selon la revendication 1, dans lequel :
le catalyseur d'alcoxyde métallique est représenté par la Formule chimique 1 ci-après :
[Formule chimique 1] X(O-R)a
dans lequel, dans la Formule chimique 1,
X est un ou plusieurs éléments sélectionnés dans le groupe constitué d'aluminium (AI), magnésium (Mg), germanium (Ge), gallium (Ga), cobalt (Co), calcium (Ca), hafnium (Hf), fer (Fe), nickel (Ni), niobium (Nb), molybdène (Mo), lanthane (La), rhénium (Re), scandium (Sc), silicium (Si), tantale (Ta), tungstène (W), yttrium (Y), zirconium (Zr) et vanadium (V).
R est un groupe alkyle ayant 1 à 10 atomes de carbone, et
a est un nombre entier de 2 ou plus.

5. Procédé de préparation d'un carbonate linéaire asymétrique selon la revendication 1, dans lequel :
le catalyseur d'alcoxyde métallique est un ou plusieurs éléments sélectionnés dans le groupe constitué de triméthoxyde d'aluminium, triéthoxyde d'aluminium, isopropoxyde d'aluminium, tert-butoxyde d'aluminium, diméthoxyde de magnésium, diéthoxyde de magnésium, isopropoxyde de magnésium, tert-butoxyde de magnésium et méthoxyde de calcium

6. Procédé de préparation d'un carbonate linéaire asymétrique selon la revendication 1, dans lequel :
une teneur du catalyseur d'alcoxyde métallique est 0,1 partie en poids ou plus et 10 parties en poids ou moins sur la base de 100 parties en poids des deux sortes de carbonates linéaires symétriques différents.

7. Procédé de préparation d'un carbonate linéaire asymétrique selon la revendication 1, dans lequel :
la réaction de transestérification est effectuée à une température de 70°C ou plus et 150°C ou moins.

8. Procédé de préparation d'un carbonate linéaire asymétrique selon la revendication 2, dans lequel :
un rapport molaire du carbonate de diméthyle et du carbonate de diéthyle est 1:0,5 à 1:1,5.

9. Procédé de préparation d'un carbonate linéaire asymétrique selon la revendication 1, consistant en outre à récupérer le carbonate linéaire asymétrique.

10. Carbonate linéaire asymétrique, comprenant un catalyseur d'alcoxyde métallique qui inclut un ou plusieurs métaux sélectionnés dans le groupe constitué d'aluminium (AI), magnésium (Mg), germanium (Ge), gallium (Ga), cobalt (Co), calcium (Ca), hafnium (Hf), fer (Fe), nickel (Ni), niobium (Nb), molybdène (Mo), lanthane (La), rhénium (Re), scandium (Sc), silicium (Si), tantale (Ta), tungstène (W), yttrium (Y), zirconium (Zr) et vanadium (V).

11. Carbonate linéaire asymétrique selon la revendication 10, dans lequel :
le catalyseur d'alcoxyde métallique est représenté par la Formule chimique 1 ci-après :
[Formule chimique 1] X(O-R)a
dans lequel, dans la Formule chimique 1 :
X est un ou plusieurs éléments sélectionnés dans le groupe constitué d'aluminium (AI), magnésium (Mg), germanium (Ge), gallium (Ga), cobalt (Co), calcium (Ca), hafnium (Hf), fer (Fe), nickel (Ni), niobium (Nb), molybdène (Mo), lanthane (La), rhénium (Re), scandium (Sc), silicium (Si), tantale (Ta), tungstène (W), yttrium (Y), zirconium (Zr) et vanadium (V) ;
R est un groupe alkyle ayant 1 à 10 atomes de carbone ; et
a est un nombre entier de 2 ou plus.

12. Carbonate linéaire asymétrique selon la revendication 10, dans lequel :
le catalyseur d'alcoxyde métallique est un ou plusieurs éléments sélectionnés dans le groupe constitué de triméthoxyde d'aluminium, triéthoxyde d'aluminium, isopropoxyde d'aluminium, tert-butoxyde d'aluminium, diméthoxyde de magnésium, diéthoxyde de magnésium, isopropoxyde de magnésium, tert-butoxyde de magnésium et méthoxyde de calcium.

13. Carbonate linéaire asymétrique selon la revendication 10, dans lequel :
une teneur du catalyseur d'alcoxyde métallique est 0,01 partie en poids ou plus et 5 parties en poids ou moins sur la base de 100 parties en poids des deux sortes de carbonates linéaires symétriques différents.

14. Carbonate linéaire asymétrique selon la revendication 10, dans lequel :
le carbonate linéaire asymétrique est le carbonate d'éthylméthyle.
